(19) Europäisches Patentamt
European Patent Office
Office européen des brevets

(11) Veröffentlichungsnummer : **0 087 047**
**B1**

(12) # EUROPÄISCHE PATENTSCHRIFT

(45) Veröffentlichungstag der Patentschrift : **07.08.85**

(51) Int. Cl.⁴ : **C 07 C125/065**

(21) Anmeldenummer : **83101121.8**

(22) Anmeldetag : **07.02.83**

(54) **Verfahren zur Herstellung von N- substituierten Monourethanen.**

(30) Priorität : **18.02.82 DE 3205891**

(43) Veröffentlichungstag der Anmeldung : **31.08.83 Patentblatt 83/35**

(45) Bekanntmachung des Hinweises auf die Patenterteilung : **07.08.85 Patentblatt 85/32**

(84) Benannte Vertragsstaaten : **BE DE FR GB IT NL**

(56) Entgegenhaltungen :
**EP-A- 0 018 581**
**EP-A- 0 060 476**

(73) Patentinhaber : **BAYER AG**
**Konzernverwaltung RP Patentabteilung**
**D-5090 Leverkusen 1 Bayerwerk (DE)**

(72) Erfinder : **Penninger, Stefan, Dr.**
**Pommernallee 5**
**D-4047 Dormagen (DE)**
Erfinder : **Knöfel, Hartmut, Dr.**
**Dülmener Weg 21**
**D-5068 Odenthal (DE)**
Erfinder : **Hammen, Günter, Dr.**
**Goethestrasse 67**
**D-4047 Dormagen 1 (DE)**
Erfinder : **Heitkämper, Peter, Dr.**
**Fliederweg 14**
**D-4047 Dormagen 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 087 047 B1

**Beschreibung**

Die vorliegende Erfindung betrifft ein Verfahren zur Herstellung von N-substituierten, aliphatischen oder aromatischen Monourethanen durch Umsetzung von primären Aminen mit N-Aryl-O-alkylurethanen in Gegenwart von hochsiedenden Alkoholen.

Die phosgenfreie Herstellung von Urethanen und deren anschließende thermische Spaltung unter Bildung der entsprechenden Isocyanate stellt auch bezüglich der Herstellung von Monoisocyanaten eine interessante Alternative zur bekannten Phosgenierung der den Isocyanaten zugrundeliegenden Amine dar. So können beispielsweise die in den Europäischen Offenlegungsschriften 27 952, 27 940, 28 331, 27 953, 18 581 und 18 583 bzw. in den Deutschen Offenlegungsschriften 2 917 490 bzw. 2 917 568 offenbarten Verfahren im Prinzip auch zur Herstellung von N-O-substituierten Monourethanen dienen. Nach den in diesen Veröffentlichungen dargelegte Prinzipien gelingt beispielsweise die Herstellung von einfachen, am Stickstoff aromatisch und am Sauerstoff aliphatisch substituierten Urethanen wie z. B. von N-Phenyl-O-ethyl-urethan in guten Ausbeuten. Die bekannten Verfahren des Standes der Technik sind jedoch weit weniger gut zur Herstellung N-Alkyl-, N-Cycloalkyl- oder von N-Aryl-urethanen mit elektronenanziehenden Substituenten am Arylrest geeignet, da bei Verwendung der diesen Urethanen zugrundeliegenden Monoamine bei den verfahren des Standes der Technik mit erheblich verminderten Ausbeuten gerechnet werden muß. Dies gilt insbesondere auch für die dem nachstehend näher beschriebenen erfindungsgemäßen verfahren nächstliegenden Verfahren des Standes der Technik (EP-OS-18 581, 18 583, 27 952 bzw. DE-OS-2 917 490 bzw. 2 917 568).

Es war daher die der Erfindung zugrundeliegende Aufgabe, ein neues Verfahren zur Verfügung zu stellen, welches die Herstellung von Monourethanen, die vorzugsweise am Stickstoff aliphatisch oder aromatisch substituiert sind und am Sauerstoff aliphatische Reste tragen, zur Verfügung zu stellen, welches nicht mit den Nachteilen der Verfahren des Standes der Technik behaftet ist. Das neue Verfahren sollte insbesondere dazu geeignet sein, neben den genannten, am Stickstoff aliphatisch substituierten Urethanen auch solche am Stickstoff aromatisch-substituierte Urethane in guten Ausbeuten herzustellen, welche am Arylsubstituenten elektronenanziehende Reste, insbesondere Halogenatome aufweisen.

Diese Aufgabe konnte überraschenderweise durch das nachstehend näher beschriebene erfindungsgemäße Verfahren gelöst werden, bei welchem man primäre Amine in Gegenwart von hochsiedenden Alkoholen mit N-Aryl-O-alkylurethanen unter Abspaltung von Arylamin zur Reaktion bringt. Die mit dem erfindungsgemäßen Verfahren erreichten Vorteile, insbesondere gegenüber den Verfahren der zuletztgenannten Vorveröffentlichungen, sind überraschend, da beim erfindungsgemäßen Verfahren, im Unterschied zu den Verfahren dieser Veröffentlichungen, die sich N-unsubstituierter Urethane als Ausgangsmaterialien bedienen, kein gasförmiges Ammoniak sondern vergleichsweise schwer flüchtiges Arylamin entsteht, welches aus dem Reaktionsgemisch entfernt werden muß, und dennoch ein nahezu vollständiger Umsatz des eingesetzten Amins unter praktisch ausschließlicher Bildung der angestrebten Verfahrensprodukte innerhalb vergleichsweise kurzer Reaktionszeiten möglich ist.

Gegenstand der Erfindung ist ein Verfahren zur Herstellung von N-substituierten O-Alkyl-urethanen, dadurch gekennzeichnet, daß man a) bei 1 013 mbar nicht destillierbare oder mindestens 20° über dem Siedepunkt des dem N-Aryl-O-alkylurethan c) entsprechenden Arylamins liegenden Siedepunkt aufweisende primäre Monoamine der Formel

$$R^1NH_2$$

in Gegenwart mindestens eines hochsiedenden Alkohols b), der bei 1 013 mbar einen mindestens 20 °C oberhalb des Siedepunkts des dem N-Aryl-O-Alkylurethan c) entsprechenden Arylamins liegenden Siedepunkt aufweist der Formel

$$R^2—OH$$

mit c) N-Aryl-O-alkylurethanen der Formel

$$R^3—NHCOO—R^4$$

bei erhöhter Temperatur zur Reaktion bringt und das sich spontan bildende Arylamin $R^3—NH_2$ kontinuierlich durch Destillation aus dem Reaktionsgemisch entfernt, wobei in diesen Formeln

$R^1$ für einen gesättigten, unsubstituierten oder Halogen- insbesondere Chlor-Substituenten aufweisenden aliphatischen Kohlenwasserstoffrest mit 9 bis 18 Kohlenstoffatomen, einen gesättigten, gegebenenfalls Halogen- insbesondere Chlor- oder Alkyl- bzw. Chloralkyl-substituierten und/oder Methylbrücken aufweisenden cycloaliphatischen Kohlenwasserstoffrest mit insgesamt 6 bis 25 Kohlenstoffatomen oder einen gegebenenfalls Halogen- insbesondere Chlor- oder Alkyl- bzw. Chloralkyl-substituierten und/oder Methylbrücken aufweisenden aromatischen Kohlenwasserstoffrest mit insgesamt 6 bis 25 Kohlenstoffatomen steht,

$R^2$ für einen gegebenenfalls Ethergruppen aufweisenden aliphatischen Kohlenwasserstoffrest mit

insgesamt 6 bis 18 Kohlenstoffatomen, einen cycloaliphatischen Kohlenwasserstofrest mit 8 bis 15 Kohlenstoffatomen oder einen araliphatischen Kohlenwasserstoffrest mit insgesamt 7 bis 18 Kohlenstoffatomen steht,

$R^3$ für einen Phenyl- oder Tolylrest steht und

$R^4$ für einen Rest steht, der bezüglich seiner Bedeutung der Definition von $R^2$ entspricht, jedoch nicht zwingend gleich $R^2$ ist.

Ausgangsmaterialien des erfindungsgemäßen Verahrens sind a) organische primäre Monoamine b) hochsiedende Alkohole mit primären oder sekundären Hydroxylgruppen und c) N-Aryl-O-alkylurethane.

Geeignete primäre Amine a) sind solche der obengenannten allgemeinen Formel wie z. B. Nonylamin, Decylamin, Decylamin, Undecylamin, Dodecylamin, Tridecylamin, Tetradecylamin, Pentadecylamin, Hexadecylamin, Heptadecylamin, Stearylamin, Cyclooctylamin, 4-(Trichlormethyl)-cyclohexylamin, 2-Chlor-cycloheptylamin, Bicyclohexyl-4-amin und dessen Isomere, 4-Aminodiphenyl und dessen Isomere, α-, β-Naphthylamin, o-, m-, p-Benzylanilin, Hexahydrobenzylanilin, Perhydrobenzylanilin, Isomere von Benzyl-toluidin, Isomere von Hexahydro- und Perhydro-benzyl-toluidin, 1-, 2-, 3-, 4-Methyl-cyclohexylamin, 3,4-Dichloranilin, 3,5-Dichloranilin, 4-Chloranilin, o-, m-, p-Toluidin und 3-Chlor-4-methylanilin.

Für das erfindungsgemäße Verfahren geeignete höhersiedende Alkohole b) sind solche der obengenannten allgemeinen Formel, die primäre oder sekundäre, vorzugsweise primäre Hydroxylgruppen und unter Normaldruk einen Siedepunkt von mindestens 190 °C aufweisen wie z. B. 1-Octanol, 1-Nonanol, 1-Decanol, 1-Undecanol, 1-Dodecanol, 1-Tetradecanol, 1-Hexadecanol, 1-Octadecanol, 2-Nonanol, Diethylenglykolmonoethylether, Benzylalkohol, 2,4,5-Trimethyl-cyclohexanol, 3-Methylbenzylalkohol, Cyclooctanol, 1,2,4-Trimethyl-cyclohexan-5-ol, oder 4-Methylbenzylalkohol. Beliebige Gemische derartiger Alkohole können selbstverständlich ebenfalls eingesetzt werden.

Für das erfindungsgemäße Verfahren geeignete N-Aryl-O-alkylurethane c) sind solche der obengenannten allgemeinen Formel wie z. B. N-Phenyl- (oder N-Tolyl-)-O-1-octyl-, -1-decyl-, -1-undecyl-, -1-dodecyl-, -1-tetradecyl-, -1-hexadecyl-, -1-octadecyl-, -2-nonyl-, -ethoxy-ethoxy-ethyl-, -2,4,5-trimethylcyclohexyl-, -3-methylbenzyl- oder -4-methylbenzyl-urethan eingesetzt. Der dem Urethan zugrundeliegende Alkohol weist primäre oder sekundäre, vorzugsweise primäre Hydroxylgruppen auf.

Beim erfindungsgemäßen Verfahren können also auch solche Urethane c) der genannten allgemeinen Formel eingesetzt werden, deren Rest $R^4$ sich von einem unter Normaldruck unterhalb 190 °C siedenden Alkohol ableitet. Dies bedeutet insbesondere, daß beim erfindungsgemäßen Verfahren auch solche Urethane c) der genannten allgemeinen Formel eingesetzt werden können, für welcher $R^4$ für einen primären oder sekundären, vorzugsweise primären gesättigten aliphatischen Kohlenwasserstoffrest mit 1 bis 4 Kohlenstoffatomen steht. Im Falle der Verwendung derartiger Urethane c) findet bei der Durchführung des erfindungsgemäßen Verfahrens vor, während und/oder nach der Abspaltung des Arylamins $R^3$—$NH_2$ eine Verdrängung des niedrigsiedenden Alkohols R4—OH durch den hochsiedenden Alkohol $R^2$—OH statt, so daß der niedrigsiedende Alkohol vor und/oder zusammen mit und/oder nach dem Arylamin aus dem Reaktionsgemisch abdestilliert. Im allgemeinen läuft diese Umurethanesierung schneller als die erfindungsgemäße Umsetzung statt, so daß diese Variante auf eine Herstellung in situ des Urethans c) auf Basis eines hochsiedenden Alkohols hinausläuft.

Die Urethane c) können grundsätzlich nach allen bekannten Verfahren des Standes der Technik hergestellt werden, beispielsweise durch die bekannte Umsetzung von Arylamin $R^3$—$NH_2$ mit Harnstoff und Alkohol $R^4$—OH.

Die beim erfindungsgemäßen Verfahren bevorzugt einzusetzenden Urethane c) auf Basis von Alkoholen $R^4$—OH mit einem bei Normaldruck mindestens bei 190 °C liegenden Siedepunkt können beispielsweise auch vor der Durchführung des erfindungsgemäßen Verfahrens durch Umurethanisierung von bezüglich des N-Substituenten entsprechenden Urethanen auf Basis niedrigsiedender Alkohole der beispielhaft genannten Art hergestellt werden. Zu dieser Umurethanisierung genügt es, das letztgenannte Urethan beispielsweise zusammen mit einem 0,1- bis 5-molaren Überschuß eines Alkohols $R^2$—OH auf ca. 130 bis 300 °C zu erhitzen und den sich bildenden niedrigsiedenden Alkohol kontinuierlich durch Destillation zu entfernen. Die hierzu benötigten N-Aryl-O-alkylurethane auf Basis niedrigsiedender Alkohole ihrerseits können nach den bekannten Methoden des Standes der Technik erhalten werden.

Bei der Durchführung des erfindungsgemäßen Verfahrens kommen die Reaktionspartner a), b) und c) im allgemeinen in einem Molverhältnis von Alkohol b) zu Amin a) von 1 : 1 bis 50 : 1, vorzugsweise 2 : 1 bis 15 :, 1 bzw. einem Molverhältnis von Urethan c) zu Amin a) von 1 : 1 bis 5 : 1, vorzugsweise 1 : 1 bis 2,5 : 1 entsprechenden Mengen zum Einsatz. Das Molverhältnis zwischen b) und c) liegt demzufolge im allgemeinen zwischen 1 : 1 und 10 : 1.

Bei der Durchführung des erfindungsgemäßen Verfahrens können auch Katalysatoren mitverwendet werden, obwohl dies im allgemeinen nicht erforderlich ist. Als die erfindungsgemäße Umsetzung beschleunigende Katalysatoren eignen sich beliebige Katalysatoren, die einen katalytischen Einfluß auf die Veresterung von Carbonsäuren ausüben, wie z. B. (i) unter den Reaktionsbedingungen inerte anorganische oder organische Basen, (ii) Lewis-Säuren oder (iii) Salze bzw. Komplexverbindungen, insbesondere Chelate von Übergangsmetallen.

Beispiele geeigneter Katalysatoren der Gruppe (i) sind tert.-Amine wie Triisopentylamin, Diethylbenzylamin, N,N-Dimethyl-benzylamin, Hexahydrodimethylanilin, N-Ethylpiperazin, Diethyl-(2-methoxypropyl)-amin, 2-(Diethylaminoethyl)-phenylether, Oxethylmorpholin, N-(2-Diethylaminoethyl)-benzamid, N-(2-Diethylaminoethyl)-propionamid, 1,4-Diaza-(2,2,2)-bicyclooctan, N,N-Dimethyl-4-aminopyridin, 1-Azabicycloheptane, 1-Azabicyclooctane, gesättigte polyheterocycliche Amine, wie 3-Methylconidin, 1-Azabicyclo-(3,2,1)-octan und Chinuclidine, Alkoholate wie z. B. Natriummethylat, Natriumethylat, Kalium-t-butylat, Titantetrabutylat, Phenolate wie z. B. Natriumphenolat oder Titantetraphenolat, anorganische Basen wie Berylliumhydroxid und Natrium-, Kalium-, Lithium-, Magnesium-, Barium- oder Calciumhydroxid, basische Alkalisalze wie Natriumcarbonat, Natriumsulfid, Kaliumcarbonat oder Trinatriumphosphat sowie Alkalisalze von Fettsäuren oder Sulfonsäuren.

Geeignete Katalysatoren (ii) sind beispielsweise Lewis-Säuren wie Eisen-II-chlorid, Eisen-III-chlorid, Zinkchlorid, Zinn-II-chlorid, Zinn-IV-chlorid, Aluminiumchlorid, Zinkcyanid, Thalliumtrichlorid, Bortrifluorid oder Bortrifluoridetherat.

Geeignete Katalystoren der Gruppe (iii) sind beispielsweise Salze von Übergangsmetallen, soweit sie nicht bereits in die Gruppe (ii) fallen, sowie Komplexverbindungen, insbesondere Chelate dieser Metalle wie Kobalt-, Mangan- oder Bleinaphthenate, Eisenoleate oder -carbonyle, Acetylacetonate von Eisen, Nickel, Kobalt, Zink, Blei, Aluminium, Mangan, Magnesium, Molybdän, Titan, Thorium, Zirkon oder Vanadium, Bis-(dibenzoylmethan)-kupfer, Bis-(ethylacetoacetat)-kupfer, -eisen, Koordinationsverbindungen von Titan, Zirkon, Hafnium, Thorium und Mangan mit β-Diketonen, β-Ketoestern und β-Hydroxyaldehyden, Dibutylzinndilaurat, Dibutylzinndiacetat, Di-(2-ethylhexyl)-zinnoxid, Dioctylzinnoxid, Zinnsalze von $C_1$-$C_{20}$-Carbonsäuren wie Zinn-(II)-naphthenat, -hexoat, -palmitat, -stearat oder -dimethylvalerat, Acetate, Chloride, Sulfate oder Octoate des zwei- oder dreiwertigen Cobalts, des ein- oder zweiwertigen Kupfers, des Zinks oder des zweiwertigen Bleis.

Besonders gut geeignete Katalysatoren sind beispielsweise Zinkchlorid, Zinkacetat, Zinkoctoat, Zinkoxid, Zinkcyanid, Dibutylzinnoxid, Dibutylzinndiethylat, Dimethylzinndichlorid, Zinn-II-chlorid, Zinn-IV-chlorid, Dibutylzinndilaurat, Kobalttrriacetat, Kobalttrichlorid, Kobalttrioctoat, Kupfer-(II)-acetat, Kupfer-(I)-chlorid, Kupfer-(II)-sulfat, Bleiacetat oder Bleichlorid.

Die Menge des jeweils eingesetzten Katalysators liegt im allgemeinen zwischen 1 ppm und 20 Gew.-%, bevorzugt zwischen 100 ppm und 5 Gew.-%, bezogen auf die Summe der Ausgangsmaterialien a), b) und c). Man wird in der Praxis selbstverständlich bestrebt bleiben, die Konzentration der Katalysatoren möglichst gering zu halten. Die optimale Konzentration hängt von der Art der Ausgangsmaterialien und der Aktivität des jeweiligen Katalysators ab und kann in einem einfachen Vorversuch bestimmt werden.

Insbesondere im Falle der Herstellung von Monourethanen, denen nicht durch Destillation zu reinigende Monoisocyanate zugrundeliegen, werden diese erfindungsgemäß vorzugsweise ohne Mitverwendung von Katalysatoren hergestellt, um die Bildung von mit derartigen Katalysatoren verunreinigten Monoisocyanaten aus den erfindungsgemäßen Verfahrensprodukte zu vermeiden. Die erfindungsgemäße Umsetzung wird im allgemeinen im Temperaturbereich von 180 bis 300 °C, vorzugsweise 200 bis 250 °C bei einem Druck von 0,1 bis 1 500, vorzugsweise 10 bis 1 000 mbar, insbesondere 200 bis 800 mbar, d. h. vorzugsweise bei Unterdruck durchgeführt. Der Druck wird vorteilhafterweise so eingestellt, daß Arylamin $R^3$—$NH_2$ oder ein Gemisch aus Arylamin $R^3$—$NH_2$ und Alkohol $R^2$—OH sowie gegebenenfalls Alkohol $R^4$—OH und gegebenenfalls geringen Mengen Amin $R^1$—$NH_2$, jedoch vorzugsweise reines Arylamin $R^3$—$NH_2$ über eine wirksame Kolonne in eine Vorlage destilliert.

Die erfindungsgemäße Umsetzung ist im allgemeinen nach einer Reaktionszeit von 1 bis 20, vorzugsweise 2 bis 10 und insbesondere 3 bis 6 Stunden beendet. Vorzugsweise wird das erfindungsgemäße Verfahren dergestalt durchgeführt, daß man die Reaktionskomponenten b) und c) in den obengenannten Mengenverhältnissen vorlegt und die Reaktionskomponente a), gegebenenfalls gelöst in Alkohol $R^2$—OH, unter innigem Durchmischen in die Vorlage eindosiert. Die flüchtigen Bestandteile des Reaktionsgemischs bzw. die flüchtigen Folgeprodukte der erfindungsgemäßen Umsetzung, insbesondere das entstehende Arylamin $R^3$—$NH_2$ werden kontinuierlich durch Destillation aus dem Reaktionsgemisch entfernt, so daß sich das Reaktionsgleichgewicht in Richtung auf das angestrebte Verfahrensprodukt verschiebt. Hierdurch wird ein nahezu quantitativer Umsatz des Monoamins a) gewährleistet. Nach Beendigung der erfindungsgemäßen Umsetzung kann der im Überschuß vorliegende Alkohol b) durch Vakuumdestillation, vorzugsweise im Dünnschichtverdampfer vom Verfahrensprodukt entfernt werden.

Beim erfindungsgemäßen Verfahren entstehen als Verfahrensprodukte N,O-substitutuierte Monourethane der Formel

$$R^1\text{—NHCOO—}R^2$$

in welcher

$R^1$ und $R^2$ die obengenannte Bedeutung haben.

Im Falle der Verwendung von Urethanen c) auf Basis von hochsiedenden Alkoholen, die mit dem Alkohol $R^2$—OH nicht identisch sind, entstehen beim erfindungsgemäßen Verfahren Monourethane der zuletzt genannten allgemeinen Formel, in denen teilweise der Rest $R^2$ durch den von diesem Rest $R^2$ verschiedenen Rest $R^4$ ersetzt ist.

Das erfindungsgemäße Verfahren verläuft im Falle der Verwendung von Urethanen c), deren Alkoholkomponente dem Alkohol b) entspricht nach folgender Reaktionsgleichung :

$$R^1NH_2 + R^3\text{—NHCOO—}R^2 \xrightarrow{R^2\text{—OH}} R^1\text{—NHCOOR}^2 + R^3\text{—NH}_2$$

Im Falle der Verwendung von Urethanen c) auf Basis von leicht flüchtigen Alkoholen $R^4$—OH verläuft die erfindungsgemäße Umsetzung nach der nachstehenden Gleichung :

$$R^1NH_2 + R^3—NHCOO—R^4 + R^2—OH \rightarrow R^1NHCOOR^2 + R^3—NH_2 + R^4—OH$$

Es muß als überraschend bezeichnet werden, daß beim erfindungsgemäßen Verfahren die Bildung von unerwünschten N,N'-disubstituierten Harnstoff praktisch vollständig unterbleibt, obwohl die erfindungsgemäße Umsetzung bei einer Temperatur erfolgt, bei welcher normalerweise Urethane in die ihnen zugrundeliegenden Isocyanate und Alkohole gespalten werden, so daß damit gerechnet werden mußte, daß sich das neben der Aminkomponente a) vorliegende Isocyanat $R^1NCO$ mit dem primären Amin a) zu unerwünschtem N,N'-disubstituierten Harnstoff vereinigt. Diese unerwünschte Nebenreaktion wird jedoch praktisch nicht beobachtet.

Die erfindungsgemäßen Verfahrensprodukte können mit niedrig-siedenden Alkoholen mit einem bei Normaldruck unterhalb 140 °C liegenden Siedepunkt, beispielsweise mit $C_1$-$C_4$-Alkanolen der Formel

$$R^5—OH$$

in welcher

$R^5$ für einen aliphatischen Kohlenwasserstoffrest mit 1 bis 4 Kohlenstoffatomen steht,

unter Druck bei 120 bis 300 °C in Monourethane der Formel

$$R^1—NHCOOR^5$$

umurethanisiert werden. Hierzu wird der Alkohol $R^5$—OH im allgemeinen in einer Menge von 5 bis 60, vorzugsweise 10 bis 20 Mol Alkohol pro Urethangruppe eingesetzt. Nach einer Reaktionszeit von 0,5 bis 10, vorzugsweise 1 bis 4 Stunden, dampft man den niedrigsiedenden Alkohol ab und destilliert anschließend bei 0,1 bis 5 mbar den freigesetzten hochsiedenden Alkohol aus dem Reaktionsgemisch ab. Diese Umurethanisierung ist im Detail beispielsweise in der Europäischen Patentanmeldung 80 106 250.6 beschrieben.

Die so erhaltenen Monourethane der Formel

$$R^1—NHCOOR^5$$

können dann in an sich bekannter Weise thermisch in organische Monoisocyanate der Formel

$$R^1NCO$$

und Alkohole der Formel

$$R^5—OH$$

gespalten werden.

Die in den nachfolgenden Beispielen gemachten Prozentangaben beziehen sich auf Gewichtsprozente.

## Beispiel 1

In einem 2 l Vierhalskolben mit Rührer, beheizbarem Tropftrichter, Füllkörperkolonne werden 305 g (1 Mol) N-Phenyl-O-dodecylurethan und 558 g Dodecanol vorgelegt und unter Rühren auf 240 °C erhitzt. Bei einem Druck von 800 mbar tropft man innerhalb von 50 Minuten 185 g (1 Mol) geschmolzenes Dodecylamin zu und destilliert sich bildendes Anilin über die Kolonne ab. Man rührt noch weitere 3 Stunden unter destillativer Entfernung von Anilin, wobei das Vakuum auf 460 mbar gesteigert wird.

Laut Gelchromatographie entstehen 377 g (95 % d. Th.) N-Dodecyl-O-dodecylurethan.

## Beispiel 2

In der unter Beispiel 1 beschriebenen Apparatur erhitzt man 305 g (1 Mol) N-Phenyl-O-dodecylurethan und 558 g Dodecanol unter Rühren auf 240 °C. Bei 800 mbar tropft man 269 g (1 Mol) Stearylamin innerhalb von 1 Stunde zu und destilliert entstehendes Anilin ab. Man rührt noch 4,5 Stunden bei gleicher Temperatur, erhöht das Vakuum stetig auf 420 mbar und destilliert in dieser Zeit 89,8 g (97 % d. Th.) Anilin ab. Laut Gelchromatographie befinden sich im Rohprodukt 461 g (96 % d. Th.) N-Stearyl-O-dodecylurethan.

## Beispiel 3

In einem 2 l Dreihalskolben mit Rührer, Innenthermometer und Füllkörperkolonne erhitzt man 162 g

(1 Mol) 3,4-Dichloranilin, 381,2 g (1,25 Mol) N-Phenyl-O-dodecylurethan und 372 g Dodecanol unter Rühren auf 240 °C und destilliert bei einem Vakuum von 800 mbar das bei der Reaktion entstehende Anilin ab. Nach einer Reaktionszeit von 6,75 Stunden, während der das Vakuum kontinuierlich auf 300 mbar erhöht wird, werden im Destillat 84,8 g Anilin (91 % d. Th.) und 2,8 g 3,4-Dichloranilin nachgewiesen. Das Rohprodukt enthält laut Hochdruckflüssigkeitschromatogramm 337,3 g (92 % d. Th.) N-(3,4-Dichlorphenyl)-O-dodecylurethan.

Beispiel 4

In der unter Beispiel 1 beschriebenen Apparatur werden 381,2 g (1,25 Mol) N-Phenyl-O-dodecylurethan und 372 g Dodecanol auf 240 °C erhitzt. Danach tropft man innerhalb von 1 Stunde unter Rühren 127,5 g (1 Mol) geschmolzenes p-Chloranilin zu. Bei einem Vakuum von 800-335 mbar werden im Verlauf von 5,5 Stunden 84,6 g (91 % d. Th.) Anilin und 18,0 g p-Chloranilin abdestilliert. Im Rohprodukt befinden sich laut Hochdruckflüssigkeitschromatographie 264,5 g (91 % d. Th.).

Beispiel 5

In der unter Beispiel 1 beschriebenen Apparatur werden 129,5 g (1,25 Mol) N-Phenyl-O-dodecylurethan und 372 g Dodecanol unter Rühren auf 240 °C erhitzt. Anschließend läßt man 125,9 g (0,915 Mol) 3-Chlor-4-methylanilin innerhalb von einer Stunde zutropfen und destilliert bei 600-370 mbar entstehendes Anilin ab. Nach 6 stündiger Reaktionszeit befinden sich im Destillat 75 g (88 % d. Th.) Anilin und 5,4 g 3-Chlor-4-methylanilin. Im Reaktionskolben werden mittels Hochdruckflüssigkeitschromatographie 268,5 g (87 % d. Th.) N-(3-Chlor-4-methylphenyl)-O-dodecylurethan nachgewiesen.

Beispiel 6

In der unter Beispiel 1 beschriebenen Apparatur werden 381 g (1,25 Mol) N-Phenyl-O-dodecylurethan und 183 g (1 Mol) 4-Benzylanilin in 372 g Dodecanol zur Reaktion gebracht. Nach einer Reaktionszeit von 5,25 Stunden werden bei einem Druck von 800-280 mbar 76,4 g (82 % d. Th.) Anilin und 3,1 g 4-Benzylanilin abdestilliert. Im Reaktionskolben befinden sich laut Hochdruckflüssigkeitschromatographie 299,7 g (77 % d. Th.) N-(4-Benzylphenyl)-O-dodecylurethan.

Beispiel 7

In einem 2 l Mehrhalskolben mit Rührer, Tropftrichter und Füllkörperkolonne erhitzt man unter Rühren 381,3 g (1,25 Mol) N-Phenyl-O-dodecylurethan in 372 g Dodecanol auf 240 °C und läßt bei einem Vakuum von 600 mbar innerhalb einer Stunde 107 g (1 Mol) m-Toluidin zutropfen. Man erhöht das Vakuum im Verlauf von 6,5 Stunden auf 350 mbar und destilliert in dieser Zeit 67,4 g Anilin und 30,1 g Toluidin ab. Im Reaktionskolben werden mit Hilfe der Hochdruckflüssigkeitschromatographie 210,7 g (92 % d. Th.) N-(m-Tolyl)-O-dodecylurethan nachgewiesen.

**Patentansprüche**

1. Verfahren zur Herstellung von N-substituierten O-Alkylurethanen, dadurch gekennzeichnet, daß man a) bei 1 013 mbar nicht destillierbare oder mindestens 20 °C über dem Siedepunkt des dem N-Aryl-O-alkylurethan c) entsprechenden Arylamins liegenden Siedepunkt aufweisende primäre Monoamine der Formel

$$R^1NH_2$$

in Gegenwart mindestens eines hochsiedenden Alkohols b), der bei 1 013 mbar einen mindestens 20 °C oberhalb des Siedepunkts des dem N-Aryl-O-Alkylurethan c) entsprechenden Arylamins liegenden Siedepunkt aufweist der Formel

$$R^2—OH$$

mit c) N-Aryl-O-alkylurethanen der Formel

$$R^3—NHCOO—R^4$$

bei erhöhter Temperatur zur Reaktion bringt und das sich spontan bildende Arylamin $R^3—NH_2$ kontinuierlich durch Destillation aus dem Reaktionsgemisch entfernt wobei in diesen Formeln

$R^1$ für einen gesättigten, unsubstituierten oder Halogen- insbesondere Chlor-Substituenten aufweisenden aliphatischen Kohlenwasserstoffrest mit 9 bis 18 Kohlenstoffatomen, einen gesättigten, gegebenenfalls Halogen- insbesondere Chlor- oder Alkyl- bzw. Chloralkyl-substituierten und/oder Methylenbrücken aufweisenden cycloaliphatischen Kohlenwasserstoffrest mit insgesamt 6 bis 25 Kohlenstoffatomen oder einen gegebenenfalls Halogen- insbesondere Chlor- oder Alkyl- bzw. Chloralkyl-substituierten und/oder Methylenbrücken aufweisenden aromatischen Kohlenwasserstoffrest mit insgesamt 6 bis 25 Kohlenstoffatomen steht,

$R^2$ für einen gegebenenfalls Ethergruppen aufweisenden aliphatischen Kohlenwasserstoffrest mit insgesamt 6 bis 18 Kohlenstoffatomen, einen cycloaliphatischen Kohlenwasserstoffrest mit 8 bis 15 Kohlenstoffatomen oder einen araliphatischen Kohlenwasserstoffrest mit insgesamt 7 bis 18 Kohlenstoffatomen steht,

$R^3$ für einen Phenyl- oder Tolylrest steht und

$R^4$ für einen Rest steht, der bezüglich seiner Bedeutung der Definition von $R^2$ entspricht, jedoch nicht, jedoch nicht zwingend gleich $R^2$ ist.

2. Verfahren gemäß Anspruch 1, dadurch gekennzeichnet, daß als hochsiedender Alkohol b) ein solcher verwendet wird, der der Alkoholkomponente des N-Aryl-O-alkylurethans c) entspricht.

3. Verfahren gemäß Anspruch 2, dadurch gekennzeichnet, daß man das N-Aryl-O-alkylurethan c), dessen Alkoholkomponente dem hochsiedenden Alkohol b) entspricht, in situ dadurch herstellt, daß man vor oder gleichzeitig mit der Einwirkung des Amins a) ein N-Aryl-O-alkylurethan c), dessen Alkoholkomponente einen niedrigeren Siedepunkt als der hochsiedende Alkohol b) aufweist mit überschüssigen Mengen eines solchen Alkohols $R^2$—OH bei erhöhter Temperatur zur Reaktion bringt und den sich hierbei spontan bildenden niedriger siedenden Alkohol durch Destillation aus dem Reaktionsgemisch entfernt.

4. Verfahren gemäß Anspruch 1 bis 3, dadurch gekennzeichnet, daß man die Umsetzung zwischen den Reaktionspartnern a), b) und c) innerhalb des Temperaturbereichs von 180° bis 300 °C durchführt.

5. Verfahren gemäß Anspruch 1 bis 4, dadurch gekennzeichnet, daß man die Umsetzung zwischen a), b) und c) bei Unterdruck durchführt.

6. Verfahren gemäß Anspruch 1 bis 5, dadurch gekennzeichnet, daß man ein Gemisch der Reaktionskomponenten b) und c) im Molverhältnis b : c von 1 : 1 bis 10 : 1 vorlegt und auf die Reaktionstemperatur erhitzt und man die Reaktionskomponente a) unter innigem Durchmischen in die Vorlage eindosiert, wobei die Gesamtmenge der Komponente a) so bemessen wird, daß auf jede primäre Aminogruppe der Komponente a) 1 bis 5 Urethangruppen der Komponente c) entfallen.

7. Verfahren gemäß Anspruch 1 bis 6, dadurch gekennzeichnet, daß man die Umsetzung zwischen a), b) und c) in Gegenwart von Katalysatoren, ausgewählt aus der Gruppe bestehend aus organischen oder anorganischen Basen, Lewis-Säuren und Salzen oder Komplexverbindungen von Übergangsmetallen durchführt.

**Claims**

1. Process for the production of N-substituted O-alkyl urethanes, characterised in that a) primary monoamines of the formula

$$R^1NH_2$$

which, at 1 013 mbar, are non-distillable or have a boiling point at least 20 °C higher than the boiling point of the arylamine corresponding to the N-aryl-O-aryl urethane c), are reacted, in the presence of at least one high-boiling alcohol b), of the formula

$$R^2—OH$$

which has, at 1 013 mbar, a boiling point at least 20 °C higher than the boiling point of the arylamine corresponding to the N-aryl-O-alkyl urethane c), with c) N-aryl-O-alkyl urethanes of the formula

$$R^3—NHCOO—R^4$$

at an elevated temperature, and the arylamine $R^3$—$NH_2$ which is spontaneously formed is continuously removed from the reaction mixture by distillation, wherein in these formula

$R^1$ represents a saturated, aliphatic hydrocarbon radical which has 9 to 18 carbon atoms and is unsubstituted or contains halogen, in particular chlorine, substituents, a saturated cycloaliphatic hydrocarbon radical which has a total of 6 to 25 carbon atoms and is optionally substituted by halogen, in particular chlorine. or alkyl, or chloroalkyl and/or contains methylene bridges, or an aromatic hydrocarbon radical which has a total of 6 to 25 carbon atoms and is optionally substituted by halogen, in particular chlorine, or alkyl or chloroalkyl and/or contains methylene bridges,

$R^2$ represents an aliphatic hydrocarbon radical which has a total of 6 to 18 carbon atoms and optionally contains ether groups, a cycloaliphatic hydrocarbon radical with 8 to 15 carbon atoms or an araliphatic hydrocarbon radical with a total of 7 to 18 carbon atoms,

$R^3$ represents a phenyl or tolyl radical and

$R^4$ represents a radical which, with regard to its meaning, corresponds to the definition of $R^2$, but is not necessarily identical to $R^2$.

2. Process according to Claim 1, characterised in that the high-boiling alcohol b) used is one which corresponds to the alcohol component of the N-aryl-O-alkyl urethane c).

3. Process according to Claim 2, characterised in that the N-aryl-O-alkyl urethane c), the alcohol component of which corresponds to the high-boiling alcohol b), is prepared in situ by reacting, before or during the action of the amine a), an N-aryl-O-alkyl urethane c), the alcohol component of which has a lower boiling point than the high-boiling alcohol b), with excess quantities of such an alcohol $R^2$—OH, at an elevated temperature, and removing the lower-boiling alcohol spontaneously forming during this reaction from the reaction mixture, by distillation.

4. Process according to Claim 1 to 3, characterised in that the reaction between the reactants a), b) and c) is carried out within the temperature range of 180° to 300 °C.

5. Process according to Claim 1 to 4, characterised in that the reaction between a), b) and c) is carried out under reduced pressure.

6. Process according to Claim 1 to 5, characterised in that a mixture of the reaction components b) and c) is initially introduced in a molar ratio of b : c of 1 : 1 to 10 : 1 and is heated to the reaction temperature and the reaction component a) is metered into the initially introduced mixture with intensive mixing, the total quantity of component a) being such that there are 1 to 5 urethane groups of component c) to every primary amino group of component a).

7. Process according to Claim 1 to 6, characterised in that the reaction between a), b) and c) is carried out in the presence of catalysts, selected from the group consisting of organic or inorganic bases, Lewis acids and salts or complex compounds of transition metals.

**Revendications**

1. Procédé de fabrication d'O-alcoyl-uréthane N-substitués, caractérisé en ce qu'on fait réagir a) des monoamines primaires non distillables sous 1 013 mbars ou présentant un point d'ébullition se situant au moins 20 °C au-dessus du point d'ébullition de l'arylamine correspondant au N-aryl-O-alcoyl-uréthane c), et qui ont pour formule

$$R^1NH_2$$

en présence d'au moins un alcool à point d'ébullition élevé b) qui présente sous 1 013 mbars un point d'ébullition au moins 20 °C au-dessus du point d'ébullition de l'arylamine correspondant au N-aryl-O-alcoyl-uréthane c) et qui a pour formule

$$R^2—OH$$

avec c) des N-aryl-O-alcoyl-uréthanes de formule

$$R^3—NHCOO—R^4$$

à une température élevée, et en ce qu'on élimine par distillation à partir du mélange de réaction de manière continue l'arylamine $R^3$—$NH_2$ qui se forme spontanément, dans ces formules

$R^1$ représentant un radical hydrocarboné aliphatique saturé non substitué ou présentant des substituants halogène, en particulier, chlore, et ayant 9 à 18 atomes de carbone, un radical hydrocarboné cycloaliphatique saturé, éventuellement substitué par de l'halogène, en particulier du chlore, alcoyle ou chloralcoyle et/ou présentant des ponts méthylène, qui a en tout 6 à 25 atomes de carbone, ou un radical hydrocarboné aromatique éventuellement halogéno-, en particulier chloro- ou alcoyl- ou chloralcoyl-substitué et/ou présentant des ponts méthylène et qui possède en tout 6 à 25 atomes de carbone,

$R^2$ un radical hydrocarboné aliphatique présentant éventuellement des groupes éther et ayant en tout 6 à 18 atomes de carbone, un radical hydrocarboné cycloaliphatique ayant 8 à 15 atomes de carbone ou un radical hydrocarboné araliphatique ayant en tout 7 à 18 atomes de carbone,

$R^3$ représente un radical phényle ou tolyle et

$R^4$ un radical qui, quant à sa signification, correspond à la définition de $R^2$, sans pour autant être obligatoirement identique à $R^2$.

2. Procédé selon la revendication 1, caractérisé en ce qu'en tant qu'alcool b) à point d'ébullition élevé, on en utilise un qui correspond au composant alcoolique du N-aryl-O-alcoyluréthane c).

3. Procédé selon la revendication 2, caractérisé en ce qu'on prépare in situ le N-aryl-O-alcoyl-uréthane c) dont le composant alcoolique correspond à l'alcool à point d'ébullition élevé b), en faisant réagir à température élevée avant ou en même temps qu'avec l'action de l'amine a) un N-aryl-O-alcoyl-uréthane c) dont le composant alcoolique présente un point d'ébullition inférieur à celui de l'alcool b) à point d'ébullition élevé, avec des quantités exédentaires d'un tel alcool $R^2$—OH, et en ce qu'on élimine par distillation à partir du mélange de réaction l'alcool à point d'ébullition inférieur qui se forme ainsi spontanément.

4. Procédé selon les revendications 1 à 3, caractérisé en ce qu'on exécute la réaction entre les partenaires de réaction a), b) et c) dans l'intervalle de température de 180 à 300 °C.

5. Procédé selon les revendications 1 à 4, caractérisé en ce qu'on effectue la réaction entre a), b) et c) sous pression réduite.

6. Procédé selon les revendications 1 à 5, caractérisé en ce qu'on introduit d'avance un mélange des composants réactionnels b) et c) dans le rapport molaire b : c de 1 : 1 à 10 : 1 et on le chauffe à la température de réaction, et l'on ajoute le composant réactionnel a) avec mélange intime dans le réceptacle, la quantité totale du composant a) étant mesurée en sorte que, pour chaque groupe amino primaire du composant a), il y ait 1 à 5 groupes uréthane du composant c).

7. Procédé selon les revendications 1 à 6, caractérisé en ce qu'on exécute la réaction entre a), b) et c) en présence de catalyseurs choisis dans le groupe consistant en des bases organiques ou minrales, en des acides de Lewis et en des sels ou composés complexes de métaux de transition.